# NEW EUROPEAN PATENT SPECIFICATION

(11) **EP 1 365 707 B2**
(45) Date of publication and mention of the opposition decision: **11.05.2016**
(45) Mention of the grant of the patent: 14.04.2010
(21) Application number: 02704340.5
(22) Date of filing: 01.02.2002
(51) Int. Cl.: A61F 2/06

(54) **IMPLANT DELIVERY SYSTEM WITH INTERLOCK**
GEFÄSSSTÜTZEINFÜHRUNGSSYSTEM MIT VERBLOCKUNGSVORRICHTUNG
SYSTÈME DE MISE EN PLACE D'IMPLANT POURVU D'UN DISPOSITIF DE VERROUILLAGE

(30) Priority: 26.02.2001 US 795047; 17.09.2001 US 954555
(43) Date of publication of application: 03.12.2003
(60) Divisional application: 10159798.7
(73) Proprietor: Covidien LP, Mansfield, MA 02048 (US)
(72) Inventor: THOMPSON, Paul, J., Minnetonka, MN 55343 (US); LEE, Nathan, T., Golden Valley, MN 55422 (US)
(74) Representative: Gray, James
(86) International application number: PCT/US2002/003153
(87) International publication number: WO 2002/067782

(56) References cited:
- EP-A- 0 775 470
- EP-A- 0 943 302
- EP-A1- 1 000 590
- EP-A1- 1 634 546
- EP-A2- 0 657 147
- EP-A2- 1 157 673
- EP-A2- 1 212 988
- WO-A1-02/32496
- WO-A1-97/16133
- DE-C2- 3 342 798
- DE-U1- 29 904 817
- US-A- 4 580 568
- US-A- 4 617 932
- US-A- 5 035 706
- US-A- 5 261 916
- US-A- 5 480 423
- US-A- 5 534 007
- US-A- 5 702 419
- US-A- 5 728 131
- US-A- 5 769 887
- US-A- 5 824 041
- US-A- 5 843 167
- US-A- 5 954 729
- US-A- 6 004 328
- 'Shorter Oxford English Dictionary', vol. 1, 2002, OXFORD UNIVERSITY PRESS INC., NEW YORK page 940 & 1680

## Description

### Field of the Invention

This invention pertains to a stent delivery system. More particularly, this invention pertains to a delivery system for a self-expandable implant such as a stent.

### BACKGROUND OF THE INVENTION

### Description of the Prior Art

Stents are widely used for supporting a lumen structure in a patient's body. For example, stents may be used to maintain patency of a coronary artery, other blood vessels or other body lumen.

Stents are commonly metal, tubular structures. Stents are passed through a body lumen in a collapsed state. At the point of an obstruction or other deployment site in the body lumen, the stent is expanded to an expanded diameter to support the lumen at the deployment site.

In certain designs, stents are open-celled tubes that are expanded by inflatable balloons at the deployment site. This type of stent is often referred to as a "balloon expandable" stent. Other stents are so-called "self-expanding" stents. Self- expanding stents do not use balloons to cause the expansion of the stent. An example of a self-expanding stent is a tube (e.g., a coil tube or an open-celled tube) made of an elastically deformable material (e.g., a superelastic material such a nitinol). This type of stent is secured to a stent delivery device under tension in a collapsed state. At the deployment site, the stent is released so that internal tension within the stent causes the stent to self-expand to its enlarged diameter. Other self- expanding stents are made of so-called shape-memory metals. Such shape-memory stents experience a phase change at the elevated temperature of the human body. The phase change results in expansion from a collapsed state to an enlarged state.

A delivery technique for elastically deformable stents is to mount the collapsed stent on a distal end of a stent delivery system. Such a system would include an outer tubular member and an inner tubular member. The inner and outer tubular members are axially slideable relative to one another. The stent (in the collapsed state) is mounted surrounding the inner tubular member at its distal end. The outer tubular member (also called the outer sheath) surrounds the stent at the distal end.

Prior to advancing the stent delivery system through the body lumen, a guide wire is first passed through the body lumen to the deployment site. The inner tube of the delivery system is hollow throughout its length such that it can be advanced over the guide wire to the deployment site.

The combined structure (i. e., stent mounted on stent delivery system) is passed through the patient's lumen until the distal end of the delivery system arrives at the deployment site within the body lumen. The deployment system may include radiopaque markers to permit a physician to visualize positioning of the stent under fluoroscopy prior to deployment.

At the deployment site, the outer sheath is retracted to expose the stent. The exposed stent is now free to self-expand within the body lumen. Following expansion of the stent, the innertube is free to pass through the stent such that the delivery system can be removed through the body lumen leaving the stent in place at the deployment site.

An embodiment of a stent delivery system is known from EP-A-0943302. In the prior art system, the stent may prematurely deploy as the outertube is retracted. Namely, with the outer tube partially retracted, the exposed portion of the stent may expand resulting in the remainder of the stent being squeezed out of the outer tube. This can result in the stent being propelled distally beyond a desired deployment site. Also, once the stent is partially unsheathed it is sometimes determined that the stent placement needs to be adjusted. With existing systems, this is difficult since the stent has a tendency to force itself out of the sheath thereby making adjustments difficult. What is needed is a system that retains the stent on the catheter even when a majority of the stent has been exposed by retraction of the sheath. What is also needed is a system that allows a stent to be re-sheathed even after a majority of the stent has been exposed by retraction of the sheath.

EP 1000590 discloses a precursor stent and a delivery apparatus therefor which is easily recaptured and repositioned within the body.

### SUMMARY OF THE INVENTION

It is an object of the invention to provide a stent delivery system wherein a stent is delivered and deployed with increased accuracy.

The stent delivery system according to the invention is defined in claim 1.

Aspects of the invention will be discussed in more detail with reference to the drawings, wherein like reference signs represent like elements. It will be appreciated that the figures are presented for illustrative purposes and may not be used to limit the scope of protection of the appended claims.

### BRIEF DESCRIPTION OF THE DRAWINGS

Fig. 1 is a side elevation view of a stent delivery system according to the present invention;
Fig. 2A is an enlarged cross-sectional view of detail A of Fig. 1 with the stent in a compressed orientation;
Fig. 2B is an enlarged cross-sectional view of detail A of Fig. 1 with the stent in a deployed (i. e., expanded) orientation;
Fig. 3 is an enlarged cross-sectional view of detail B of Fig. 1;
Fig. 4 is an enlarged cross-sectional view of detail C;
Fig. 5 is a cross-sectional view of the inner and outer tubular members of the stent delivery system of Fig. 1 taken along section line 5-5 of Fig. 3;
Fig. 6A is a plan view of a first stent having an interlock structure that interlocks with an interlock structure of a mating collar, the stent and the collar are shown cut longitudinally and laid flat with an axial separation between the stent proximal end and the mating collar;
Fig. 6B is the view of Fig. 6A with the stent proximal end and mating collar shown interlocked;
Fig. 6C is an end view of the stent of Figs. 6A and 6B in its tubular configuration;
Fig. 7 is a laid flat, plan view of a second stent having an interlock structure that interlocks with an interlock structure of a mating collar, the collar includes rotational positioning indicators;
Fig. 8 is a laid flat, plan view of a third stent having an interlock structure that interlocks with an interlock structure of a mating collar, the collar includes rotational positioning notches;
Fig. 9 is a laid flat, plan view of a fourth stent having an interlock structure that interlocks with an interlock structure of a mating collar, the stent and the collar include a rotational alignment key and keyway;
Fig. 10 is a laid flat, plan view of a fifth stent having an interlock structure that interlocks with an interlock structure of a mating collar;
Fig. 11 is a laid flat, plan view of a sixth stent having an interlock structure that interlocks with an interlock structure of a mating collar;
Fig. 12 is a laid flat, plan view of a seventh stent having an interlock structure that interlocks with rectangular posts formed on an inner body of a catheter;
Fig. 13 is a laid flat, plan view of a eighth stent having an interlock structure that interlocks with an interlock structure of a mating collar;
Fig. 14A is a laid flat, plan view of a ninth stent having an interlock structure that interlocks with outwardly projecting line-like projections formed on the inner body of a catheter;
Fig. 14B shows the stent of Fig. 14A interlocked with the line-like projections;
Fig. 15A is a laid flat, plan view of a tenth stent having an interlock structure that interlocks with outwardly projecting posts formed on the inner body of a catheter;
Fig. 15B shows the stent of Fig. 15A interlocked with the posts; and
Figs. 16A and 16B show another delivery system that is an embodiment of the present invention.

### DETAILED DESCRIPTION

With reference now to the various drawing figures in which identical elements are numbered identically throughout, a description of a preferred embodiment of the present invention will now be provided.

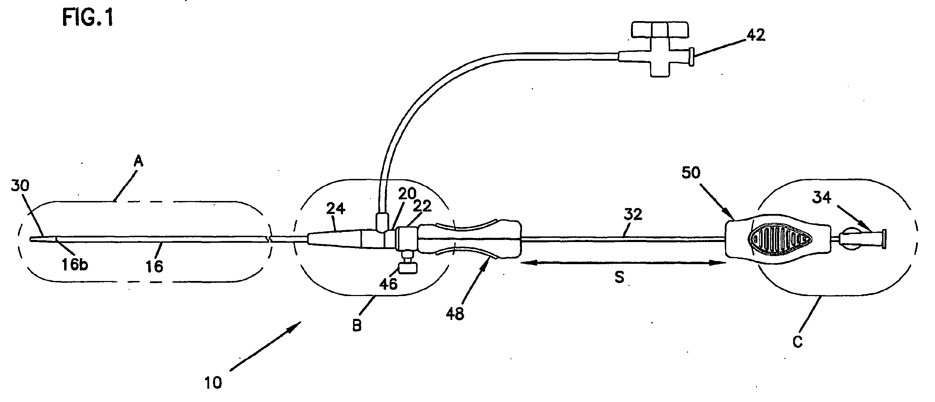

With initial references to Figs. 1 - 4, a stent delivery system 10 is shown. The stent delivery system 10 is for delivery of a stent 12 to a deployment site in a body lumen of a patient's body. Byway of non-limiting, representative example, the stent 12 may be a self-expanding stent having a construction such as that shown in U. S. Pat. No. 6,132,461. In one non-limiting embodiment, the stent can be made of a superelastic metal such as nitinol, or the like. The stent 12 may also be a coil stent or any other self-expanding stent. The stent 12 includes a proximal end 12a and a distal end 12b. Another representative stent is shown in United States patent publication US 2001-0029397 and entitled STENT.

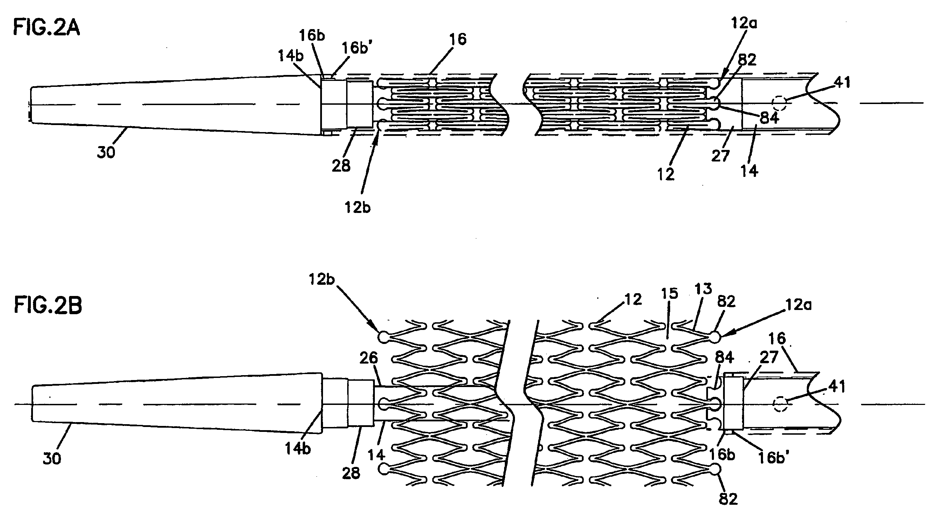

The stent 12 is carried on the stent delivery system 10 in a collapsed (or reduced diameter) state as shown in Fig. 2A. Upon release of the stent 12 from the stent delivery system 10 (as will be described), the stent 12 expands to an enlarged diameter (see Fig. 2B) to abut against the walls of the patient's lumen in order to support patency of the lumen.

The stent delivery system 10 includes an inner tubular member 14 (i.e., also referred to as an elongated member) and an outer tubular member 16. Both of the inner and outer tubular members 14 and 16 extend from proximal ends 14a, 16a to distal ends 14b, 16b.

The outer tubular member 16 is sized to be axially advanced through the patient's body lumen. The tubular member 16 is preferably sufficiently long for the distal end 16b to be placed near the deployment site in the patient's body lumen with the proximal end 16a remaining external to the patient's body for manipulation by an operator. By way of example, the outer tubular member 16 (also referred to as a sheath) may be a braid-reinforced polyester of tubular construction to resist kinking and to transmit axial forces along the length of the sheath 16. The outer tubular member 16 may be of widely varying construction to permit varying degrees of flexibility of the outer tubular member 16 along its length.

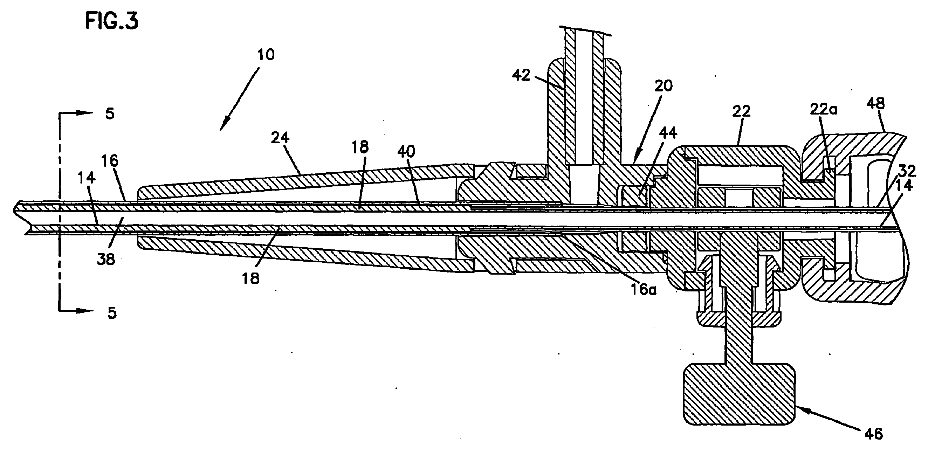

As shown in Fig. 3, the proximal end 16a of the outer tubular member 16 is bonded to a manifold housing 20. The manifold housing 20 is threadedly connected to a lock housing 22. A strain relief jacket 24 is connected to the manifold housing 20 and surrounds the outer tubular member 16 to provide strain relief for the outer tubular member 16.

The inner tubular member 14 is preferably formed of nylon but may be constructed of any suitable material. As shown in Fig. 2B, the inner tubular member 14 defines a stent attachment location 26 (i. e., a stent mounting location). The inner tubular member 14 also includes markers 27,28 that are attached to an outer surface of the inner tubular member 14(e. g., by techniques such as adhesive, heat fusion, interference fit, fasteners, intermediate members or other techniques). The attachment location 26 is positioned between the markers 27,28. The radiopaque markers 27,28 permit a physician to accurately determine the position of the stent attachment location 26 within the patient's lumen under fluoroscopic visualization. As will be described later in the specification, in some embodiments, at least one of the markers 27,28 forms a collar including a geometry that interlocks with the stent 12 to prevent axial movement of the stent 12 relative to the inner tubular member during transport and deployment of the stent 12. Materials for making the radiopaque marker should have a density suitable for visualization through fluoroscopic techniques. Exemplary materials comprise tanalum, platinum, gold, tungsten and alloys of such metals. In some embodiments, the markers can be coated with a radiopaque material or filled with a radiopaque filler.

A tapered and flexible distal tip member 30 is secured to the distal end 14b of the inner tubular member 14. The highly flexible distal tip member 30 permits advancement of the stent deployment system 10 through the patient's lumen and minimizes trauma to the walls of the patient's lumen. As shown in Fig. 2B, the inner tubular member 14 preferably extends completely through the stent 12 when the stent 12 is mounted at the attachment location 26.

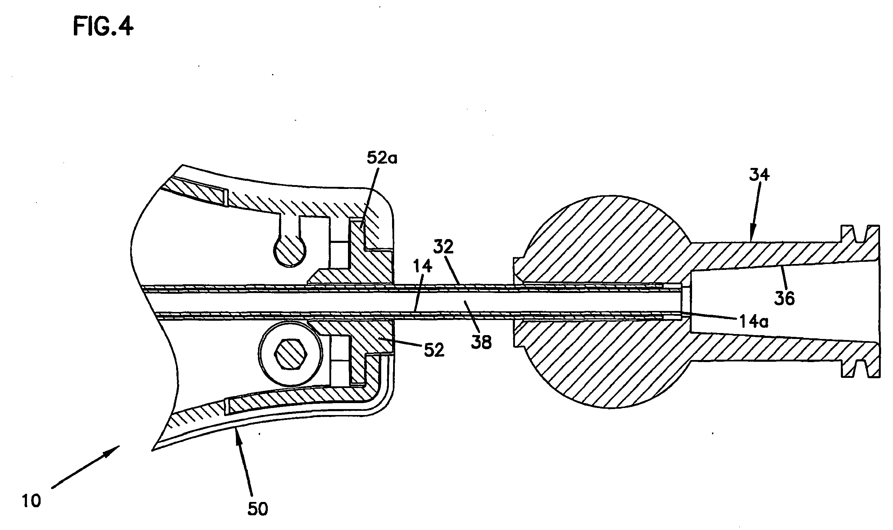

As best shown in Figs. 3 and 4, the inner tube 14 passes through both the manifold housing 20 and lock housing 22. A stainless steel jacket 32 surrounds and is bonded to the inner tubular member 14.

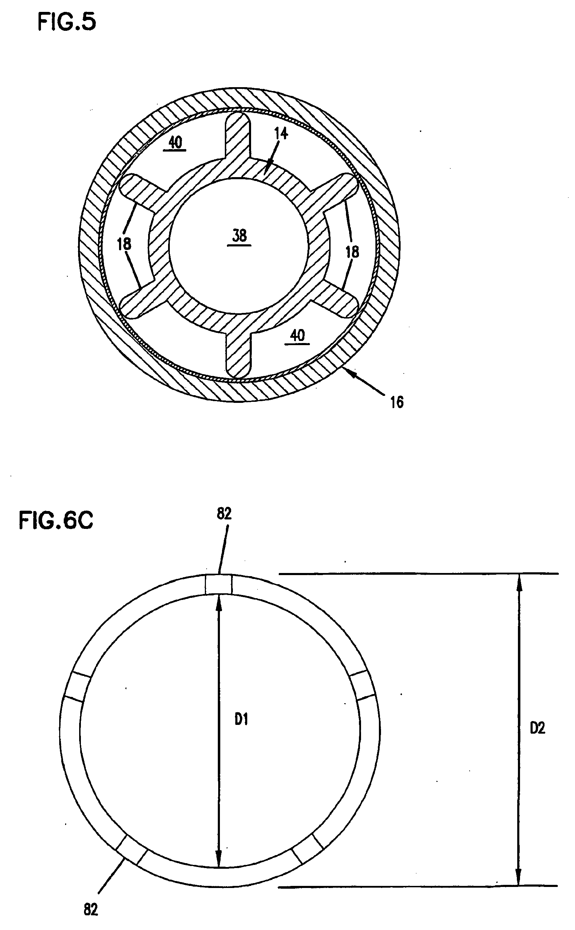

At the inner tube proximal end 14a, a port housing 34 is bonded to the stainless steel jacket 32. The port housing 34 has a tapered bore 36 aligned with an inner lumen 38 of the tubular member 14. The inner lumen 38 extends completely through the inner tubular member 14 so that the entire delivery system 10 can be passed over a guide wire (not shown) initially positioned within the patient's lumen. Opposing surfaces of the inner and outer tubular members 14 and 16, define a first lumen 40 (best seen in Fig. 5). As described in United States patent publication US 2003-0074043 and entitled STENT DELIVERY SYSTEM WITH SPACER MEMBER, splines 18 can be provided between the inner and outer tubular members 14 and 16.
As shown in Fig. 3, the manifold housing 20 carries an admission port 42 for injecting a contrast media into the interior of the manifold housing 20. The interior of the manifold housing 20 is in fluid flow communication with the first lumen 40. Discharge ports 41 (shown in Figs. 2A and 2B) are formed through the outer tubular member 16 to permit contrast media to flow from the first lumen 40 into the patient's body lumen.

As shown in Fig. 3, an O-ring 44 surrounds the stainless steel jacket 32 between the manifold housing 20 and lock housing 22. Upon threaded connection of the manifold housing 20 to the lock housing 22, the O-ring 44 compresses against the stainless steel jacket 32 in sealing engagement to prevent contrast media from flowing in any path other than through the first lumen 40.

As shown in Figs. 1 and 3, the lock housing 22 carries a threaded locking member (or lock nut) 46 which can be turned to abut the stainless steel jacket 32. The lock nut 46 can be released to free the stainless steel jacket to move axially. According, when the lock nut 46 engages the jacket 32, the jacket 32 (and attached inner tubular member 14) cannot move relative to the lock housing 22, manifold housing 20 or the outer tubular member 16. Upon release of the lock nut 46, the inner tubular member 14 and outer tubular member 16 are free to slide axially relative to one another between a transport position and a deploy position.

First and second handles 48, 50 are secured to the lock housing 22 and jacket 32, respectively. In the transport position (shown in Fig. 2A), the handles 48, 50 are spaced apart and the distal end of the outer tubular member 16 forms a sheath that covers the stent attachment location 26 to prevent premature deployment of the stent 12. When the handle 48 is pulled rearwardly toward the handle 50, the outer tubular member 16 slides rearwardly or proximally relative to the inner tubular member 14. Preferably, the outer tubular member 16 slides rearwardly a distance sufficient to fully expose the stent attachment location 26 and permit the stent 12 to freely expand toward its fully expanded diameter (see Fig. 2B). After such expansion, the stent delivery system can be proximally withdrawn through the expanded stent and removed.

As shown in Fig. 3, the first handle 48 is rotatably mounted on a flange 22a of the lock housing 22. The first handle 48 surrounds the stainless steel jacket 32 and is freely rotatable about the longitudinal axis of the jacket 32 and freely rotatable about the flange 22a. The first handle 48 is axially affixed to the lock housing 22 such that axial forces applied to the first handle 48 are transmitted through the lock housing 22 and manifold housing 20 to the outer tubular member 16 to axially move the outer tubular 16. However, rotary action of the first handle 48 about the axis of the stainless steel jacket 32 is not transmitted to the housings 20, 22 or to the outer tubular member 16 by reason of the free rotation of the first handle 48 on flange 22a.

As shown in Fig. 4, the second handle 50 is mounted on an anchor 52 that is bonded to the stainless steel jacket 32 through any suitable means (such as by use of adhesives). The anchor 52 includes a flange 52a that is radial to the axis of the stainless steel jacket 32. The second handle 50 is mounted on the flange 52a and is free to rotate on the anchor 52 about the axis of the stainless steel jacket 32. However, axial forces applied to the handle 50 are transmitted to the stainless steel jacket 32 which, being bonded to the inner tubular member 14, results in axial movement of the inner tubular member 14.

With the handle construction described above, relative axial movement between the handles 48, 50 results in relative axial movement between the inner and outer tubular members 14,16. Rotational movement of either of the handles 48, 50 does not affect rotational positioning of the inner or outer tubular members 14, 16 and does not affect axial positioning of the inner and outer tubes 14, 16.

The free rotation of the handles 48, 50 results in ease of use for a physician who may position his or her hands as desired without fear of interfering with any axial positioning of the inner and outer tubular members 14,16. The spacing between the handles 48, 50 is equal to the stroke between the transport position and the deploy position of the tubular members 14,16. As a result, the spacing permits the operator to have ready visual indication of the relative axial positioning between the inner and outer tubular members 14,16. This relative axial positioning can be fixed by engaging the lock nut 46. In any such positioning, contrast media can be injected through the admission port 42 into the chamber 40 with the contrast media flowing out of the side ports 41 into the body lumen to permit visualization under fluoroscopy.

With stent deployment systems having premounted stents of various axial lengths, the positioning of the second handle 50 on the stainless steel jacket 32 can be selected at time of assembly so that a spacing S (see Fig. 1) between the handles 48, 50 corresponds to the length of the stent 12 carried on the stent deployment system. For example, in a preferred embodiment, the spacing S is about 10 millimeters longer than the deployed length of the stent. Accordingly, the user will know that the outer tubular member 16 has been fully retracted when the handles 48, 50 have been pushed completely together to completely release the stent 12. Also, the freely rotatable handles 48, 50 are easy to hold from any angle without slippage. The lock nut 46 ensures that the stent 12 will not deploy prematurely.

A concern with existing delivery systems for self-expanding stents is control of stent delivery. For example, due to their elastic characteristics, self-expanding stents have a tendency to propel themselves axially outwardly from their restraining sheaths before the sheaths have been completely retracted. When this occurs, control of stent placement is compromised since the stent may overshoot the desired deployment site. Further, once the stent has been completely deployed, subsequent adjustment of the stent deployment location can be difficult because re-sheathing typically cannot be readily accomplished.

To address the above concerns, the delivery system 10 is preferably equipped with an interlock configuration that constrains relative axial movement between the stent 12 and the inner tube 14 until after the sheath 16 has been fully retracted. For example, when the stent 12 is mounted on the inner tube 14 and restrained in the compressed orientation by the sheath 16 as shown in Fig. 2A, a first interlock geometry (e.g., male interlock structures 82 as shown in Fig. 2A) located at the proximal end of the stent 12 interlocks with a second interlock geometry (e.g., female interlock structures 84 as shown in Fig. 2A) defined by the proximal marker 27 (also referred to as a collar). The interlock geometries remain interlocked to constrain axial movement of the stent 12 until after the sheath has been retracted beyond a predetermined location (e.g., the proximal-most end 12a of the stent 12). When the sheath 12 has been retracted beyond the predetermined location, the interlock geometry of the stent 12 is allowed to expand. As the interlock geometry of the stent expands, the interlock geometry of the stent disengages from the interlock geometry of the marker 27 thereby allowing the inner tube 14 of the catheter to be moved axially relative to the stent without interference from the interlock geometries.

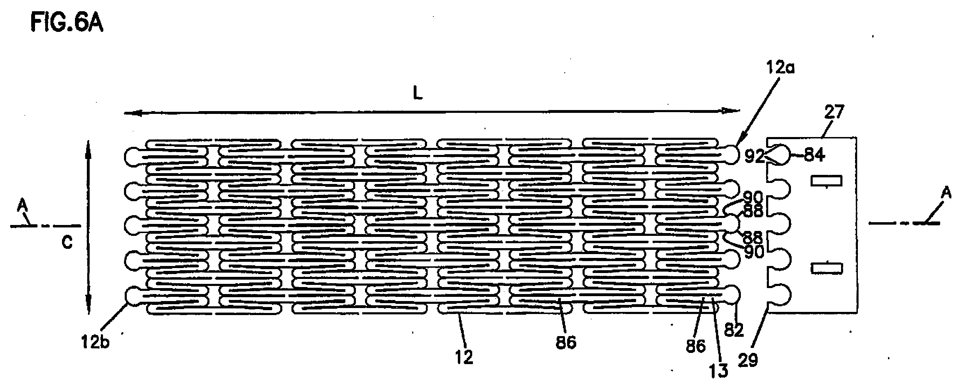

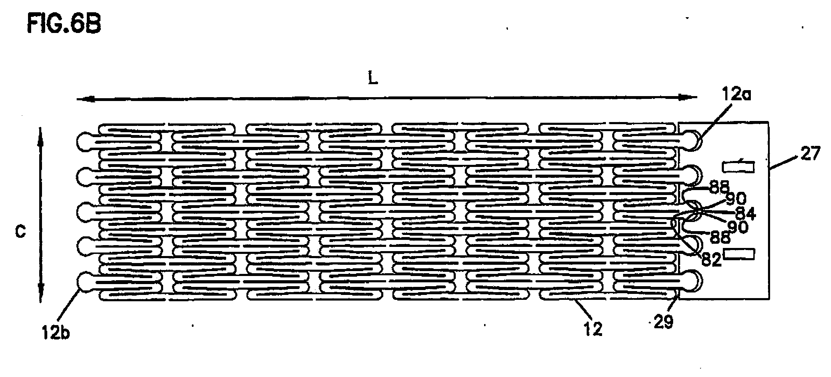

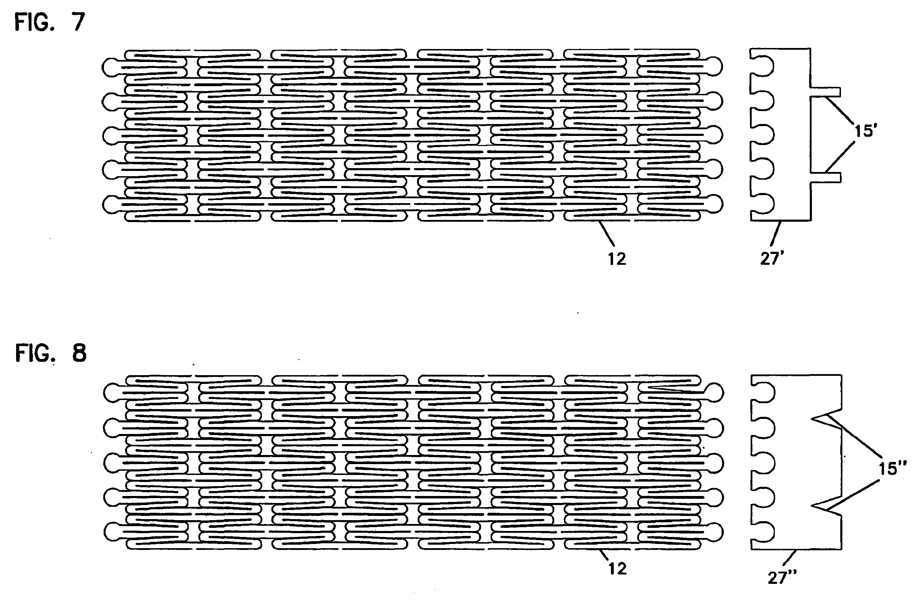

Figs. 6A and 6B illustrate the proximal end 12a of the stent 12 in relation to the marker 27 located at the proximal end of the attachment location 26. In Figs. 6A and 6B, the stent 12 and the marker 27 have been cut longitudinally and laid flat. The stent 12 has a length L and a circumference C. In Fig. 6A, the marker 27 and the stent 12 are shown disengaged from one another. In Fig. 6B marker 27 and the stent 12 are shown interlocked. In both Figs. 6A and 6B, the stent is in the reduced diameter configuration. Similarly, the stents depicted in Figs. 7-15B are shown in the reduced diameter orientation.

Referring to Fig. 6A, the stent 12 includes a plurality of struts 86 (i.e., reinforcing members). At least some of the struts 86 have free terminal ends that define the proximal and distal ends 12a and 12b of the stent 12. Male interlock structures 82 (i.e., keys) are provided at the free terminal ends of the struts 86. As shown in Fig. 6A, the male interlock structures 82 include enlargements in the form of circular projections. The circular projections include interlock portions 88 that project outwardly from the struts 86 in a circumferential direction (i.e., in a direction coinciding with the circumference C of the stent 12). The interlock portions 88 include interlock surfaces 90 that face in an axial direction. The phrase "face in an axial direction" will be understood to mean that least a vector component of the surface 90 is perpendicular with respect to a longitudinal axis A-A of the stent 12. Thus, the surface 90 need not be completely perpendicular relative to the longitudinal axis of the stent 12 to be construed as facing in an axial direction. In other words, a surface aligned at oblique angle relative to the longitudinal axis of the stent 12 shall also be construed as facing in an axial direction since such surface has a vector component that is perpendicular relative to the longitudinal axis of the stent.

As best shown schematically in Fig. 6C, the male interlock structures 82 are preferably positioned within a region defined between an inner diameter D1 and an outer diameter D2 of the stent 12. This is preferably true regardless of whether the stent 12 is in the expanded diameter orientation or the reduced diameter orientation. Preferably, at least portions of the interlock surfaces 90 are located within 5 millimeters of the proximal end 12a of the stent 12. More preferably, at least portions of the interlock surfaces 90 are located within 3 millimeters of the proximal end 12a of the stent 12. Most preferably, at least portions of the interlock surfaces 90 are located within 2 millimeters of the proximal end 12a of the stent 12.

Referring to Fig. 6A, the stent 12 includes a lumen reinforcing structure including a plurality of struts 13 adapted to define open cells 15 (best shown in Fig. 2B) when the stent 12 is deployed. Preferably, the male interlock structures 82 are located within 5 millimeter of the struts 13 that define the open cells 15. More preferably, the male interlock structures 82 are located within 4, 3 or 2 millimeters of the struts 13 that define the open cells 15. Most preferably, the male interlock structures 82 are located within 1 millimeter of the struts 13 that define the open cells 15. Because the male interlock structures 82 are located relatively close to the structure defining the open cells 15, during deployment of the stent 12, the male interlock structures 82 will expand radially outwardly simultaneously with the radial expansion of at least a portion of the cell defining structure. When the stent 12 is expanded, the interlock structures 82 are preferably maintained generally within a boundary defined by the inner and outer diameters of the cell defining portion of the stent, and preferably the interlock structures 82 are not biased or angled radially outwardly relative to the cell defining portion.

Still referring to Figs. 6A and 6B, the marker 27 has an axial distal edge 29 facing the proximal end 12a of stent 12. Female interlock structures 84 (i.e., sockets, openings, keyways, etc.) are defined by the marker 27 adjacent the edge 29. The female interlock structures 84 are configured to have a complimentary mating geometry with respect to the male interlock structures 82 of the stent 12. For example, similar to the male interlock structures 82, the female interlock structures 84 are shown having generally rounded or circular shapes. Each of the female interlock structures 84 includes interlock surfaces 92 that face in an axial direction.

The geometry of the female interlock structures 84 is selected to mate with the predetermined geometry of the stent proximal end 12a such that the stent 12 and the marker 27 can be axially coupled or interlocked when the stent 12 is compressed at the mounting location 26. When the male and female interlock structures 82 and 84 are interlocked, the interlock surfaces 90 and 92 oppose and circumferentially overlap one another (see Fig. 6B) such that the stent is restricted from distal movement relative to the marker 27.

With the specific embodiment shown, the stent 12 and collar 27 are rotary coupled such that the stent 12 and collar 27 are restricted from relative rotary motion (i.e., about axis X- X) when the stent 12 is in the collapsed state. The predetermined stent geometry of the interlock structures 82 and the complementary mating geometry of the collar 27 do not restrict relative radial motion. Namely, as the self-expanding stent 12 expands radially, the male interlock structures 82 are free to radially move out of the female interlock structures 84. After such motion, the stent 12 is no longer coupled to the collar 27 and the stent 12 and collar 27 are free to move axially, radially or transversely to one another.

With the embodiment thus described, the mating features of the stent 12 and collar 27 prevent premature discharge of the stent 12 from a stent attachment location 26. As the outer sheath 16 is retracted, the sheath distal end 16b exposes the distal end 12b of the stent 12. At this point, the exposed distal end 12b of the stent 12 is free for limited expansion restrained by the remainder of the stent 12 being covered by the sheath 16 and by the attachment of the stent proximal end 12a to the proximal marker 27.

Further retraction of the sheath 16, permits still further expansion of the stent 12. As the sheath distal end 12b approaches the stent proximal end 12a, the expansion of the stent material tends to urge the stent 12 to squeeze out of the small portion of the sheath 16 now covering the stent 12. However, this propensity is overcome by the attachment of the stent proximal end 12a to the collar 27 since any such ejection of the stent 12 would require axial separation of the stent 12 and collar 27. Such movement is prevented by the male interlock structures 82 and the female interlock structures 84.

Therefore, as long any portion of the sheath 16 overlies the male and female interlock structures 82 and 84, the proximal end 12a of the stent 12 cannot expand and cannot axially move away from the collar 27. Accordingly, the stent 12 is not released from the attachment location 26 until the physician has fully retracted the sheath 16 with the sheath distal end 16b retracted proximal to the proximal end of stent attachment location 26. The sheath distal end 16b is provided with a marker 16b' (shown in Figs. 2A and 2B) to permit visualization of the relative position of the sheath distal end 12b and the markers 27, 28 of the stent attachment location 26.

With the structure and operation thus described, the physician has greater control of the release of the stent 12. More accurate stent positioning is attained. As long as even a small portion of the sheath 16 is not fully retracted (e.g., at least 1 mm extends distally to the proximal end 12a of the stent 12) the axial position of the stent 12 can be adjusted by advancing or retracting the inner tubular member 14. Also, as long as a small portion of the sheath 16 remains covered by the sheath 16 (e.g., at least 1 mm), the stent 12 can be readily re-sheathed by moving the sheath 16 in a distal direction.

In the embodiment of Figs. 6A and 6B, the female and male interlock structures 82 and 84 have complementary mating geometries. It will be appreciated that in alternative configurations not forming part of the claimed invention, the female and male interlock structures need not have complementary/identical shapes. Instead, to provide an interlock, it is only necessary for a portion of the male interlock to be received in the female interlock such that mechanical interference or overlap between the interlocks prevents the interlocks from being axially separated. This can be accomplished without having identical mating shapes.

As described above, the interlock structure 84 of the inner tube 14 is provided on the proximal marker 27. It will be appreciated that in configurations not forming part of the claimed invention the interlock structure 84 need not be the same element as the marker but could be a separate part. As a separate part, the interlock structure could be integrally formed/connected with the exterior of the inner tube 14, connected to the outer surface of the inner tube by conventional techniques (e.g., adhesive, fasteners, fusion bonding, etc.), or be connected to the outer surface of the inner tube 14 by one or more intermediate members. Further, the embodiment of Figs. 6A and 6B shows that the interlock between the stent 12 and the tube 14 is provided at the proximal end 12a of the stent 12b. It will be appreciated that for certain embodiments, the interlock between the inner tube 14 and the stent 12 can be provided at the distal end 12b of the stent 12 (e.g., for a distally retractable sheath). Moreover, while the embodiment of Figs. 6A and 6B shows interlock structures provided at all of the proximal ends of the struts 86, the invention is not so limited. For example, in some embodiments, only some of the struts 86 may include interlock structures. While in certain embodiments it may be desirable to use only one interlock structure at the end of the stent 12, it is preferable to use at least two separate/discrete interlock structures uniformly spaced about the circumference of the stent. It is more preferable to use at least 4 separate/discrete interlock structures that are preferably uniformly spaced about the circumference of the stent.

The collar 27 may be provided with indicia to indicate to a physician the position of the collar 27 (and hence the stent 12) when the combination is in a patient's vessel and is being visualized under fluoroscopy. In the embodiment of Figs. 6A and 6B, the indicia is shown as cutouts in the collar 27. Fig. 7 shows a collar 27' halving indicia in the form of proximal projections 15' off of the proximal edge of the collar 27'. Fig. 8 shows a collar 27" having indicia in the form of triangular notches 15" defined at the proximal edge of the collar 27". In the embodiments shown, the indicia 15' and 15" are spaced apart circumferentially on their respective collars 27, 27' and 27" so that the indicia are 180 degrees apart.

In the embodiment of Figs. 6A and 6B, the pattern and shape of the male interlock structures 82 and the female interlock structures 84 are symmetrical about the stent axis X-X. As a result, the stent 12 can be affixed to the collar 27 in any one of a plurality of rotary alignments about axis X - X.

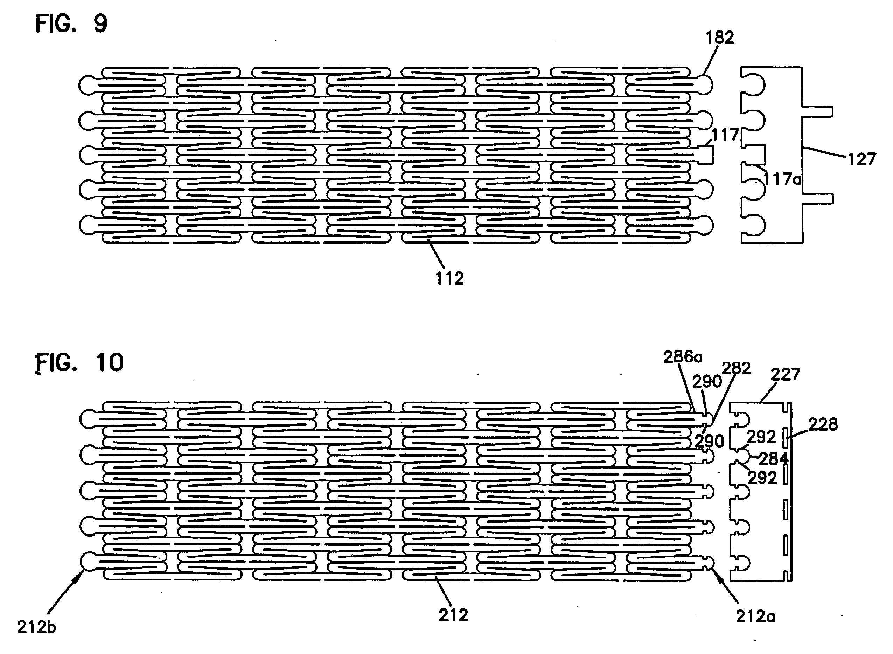

Fig. 9 illustrates an embodiment of a collar 127 and stent 112 where the symmetrical pattern is interrupted. In the example of Fig. 9, a single unique key 117 is provided (which, in the example shown, has a square geometry compared to the circular geometry of remaining male interlock structures 182). Similarly, the collar 127 has a unique keyway 117a to mate with the unique key 117. As a result, the stent 112 can only be affixed to the collar 127 in one rotary alignment.

In all of the above embodiments, once the position of a stent is fixed to a collar, a non-symmetrical stent feature (e.g., an opening for placement at a bifurcation in a vessel) can be aligned with the indicia (or, if desired, a single indicia can be provided on the collar). Therefore, a physician can easily visualize the position of any non-symmetrical stent feature.

Fig. 10 illustrates an embodiment of a stent 212 and radiopaque collar 227 having another interlock configuration. The collar 227 has circumferential slots 228 for assisting in adhesively bonding the collar 227 to the outer surface of the inner tube 14. The stent 212 has proximal and distal ends 212a and 212b. The stent also includes proximal end struts 286a having free ends at which male interlock structures 282 are formed. The male interlock structures 282 are formed by notches cut into the proximal end struts 286a. The male interlock structures 282 include axially facing interlock surfaces 290 that face in a distal direction. Preferably, the surfaces 290 are located within 5 millimeters of the proximal end 212a of the stent 212, and within 1, 2, 3, 4 or 5 millimeters of a cell defining portion of the stent.

The collar 227 includes female interlock structures 284 in the form of sockets. The sockets are partially defined by projections adapted to fit within the notches cut into the proximal end struts 286a. The projections define axially facing interlock surfaces 292 that face in a proximal direction. When the male and female interlock structures 282 and 284 are interlocked, the surfaces 290 and 292 oppose one another to prevent the male interlock structures 282 from being axially withdrawn from the female interlock structures 284.

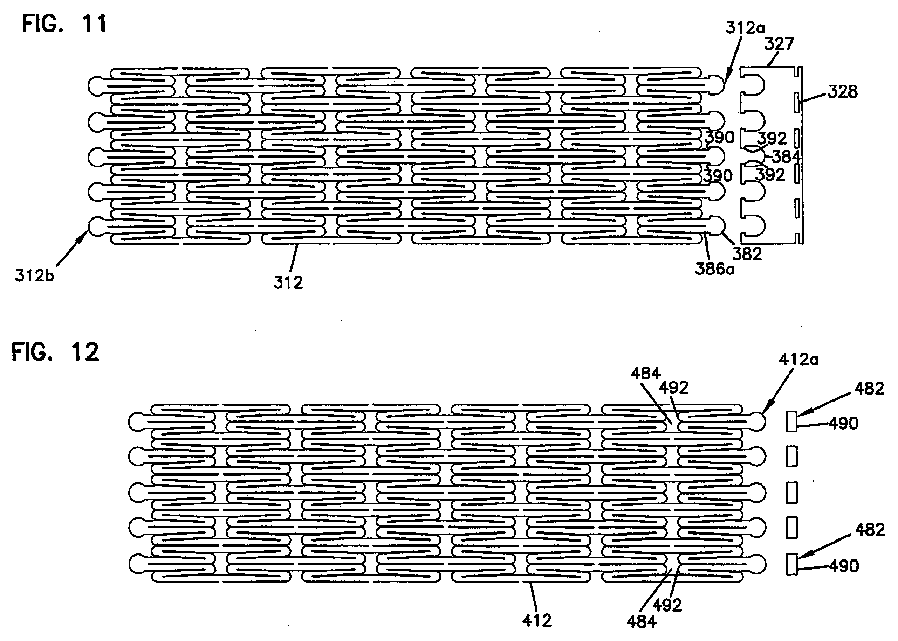

Fig. 11 illustrates an embodiment of a stent 312 and radiopaque collar 327 having another interlock configuration. The collar 327 has circumferential slots 328 for assisting in adhesively bonding the collar 327 to the outer surface of the inner tube 14. The stent 312 has proximal and distal ends 312a and 312b. The stent also includes proximal end struts 386a having free ends at which male interlock structures 382 are formed. The male interlock structures 382 are formed by enlarged heads (i.e., protuberances or keys) located at the ends of the end struts 386a. The male interlock structures 382 include axially facing interlock surfaces 390 that face in a distal direction. Preferably, the surfaces 390 are located within 5 millimeters of the proximal end 312a of the stent 312, and within 1, 2, 3, 4 or 5 millimeters of a cell defining region of the stent. The collar 327 includes female interlock structures 384 in the form of sockets. The female interlock structures 384 include axially facing interlock surfaces 392 that face in a proximal direction. When the male and female interlock structures 382 and 384 are interlocked, the surfaces 390 and 392 oppose one another to prevent the male interlock structures 382 from being axially withdrawn from the female interlock structures 384.

Fig. 12 illustrates a stent 412 including female interlock structures 484. The female interlock structures 484 preferably include distally facing interlock surfaces 492 located within 5 mm of a proximal end 412a of the stent 412 and within 1, 2, 3, 4 or 5 millimeters of a cell defining region of the stent. The female interlock structures 484 are sized to receive male interlock structures 482 in the form of rectangular posts. Preferably, the posts are connected to the outer surface of the inner tube 14 (e.g., integrally or otherwise). The posts define proximally facing interlock surfaces 490. When the female and male interlock structures 484 and 482 are coupled, the surfaces 490 and 492 engage each other to prevent distal movement of the stent 412 relative to the posts.

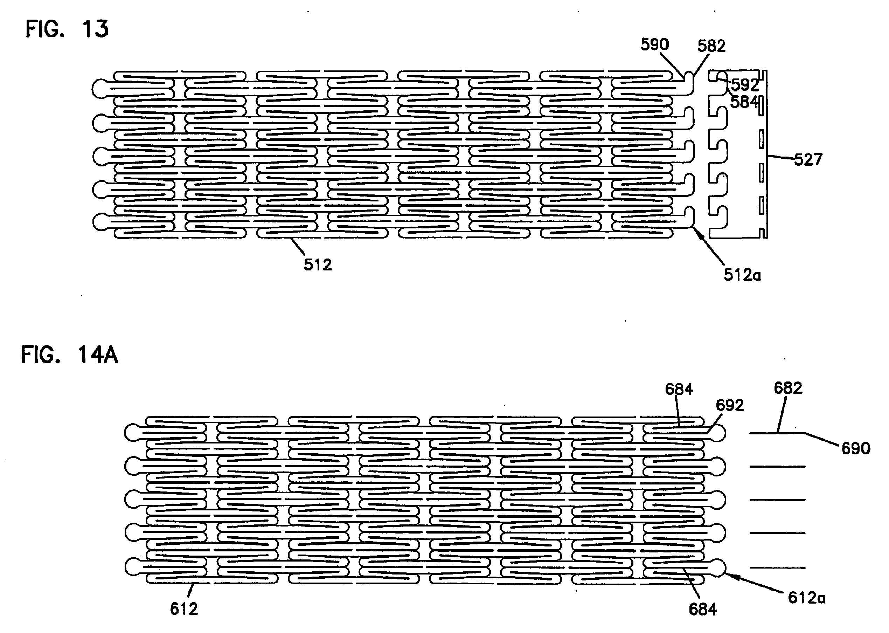

Fig. 13 illustrates an embodiment of a stent 512 including male interlock structures 582 in the form of hooks. The male interlock structures 582 preferably include distally facing interlock surfaces 590 located within 5 mm of a proximal end 512a of the stent 512 and within 1, 2, 3, 4 or 5 millimeters of a cell defining region of the stent. The male interlock structures 582 are sized to fit within female interlock structures 584 defined by a collar 527. The female interlock structures 584 define proximally facing interlock surfaces 592. When the female and male interlock structures 584 and 582 are coupled, the surfaces 590 and 592 engage each other to prevent distal movement of the stent 512 relative to the collar 527.

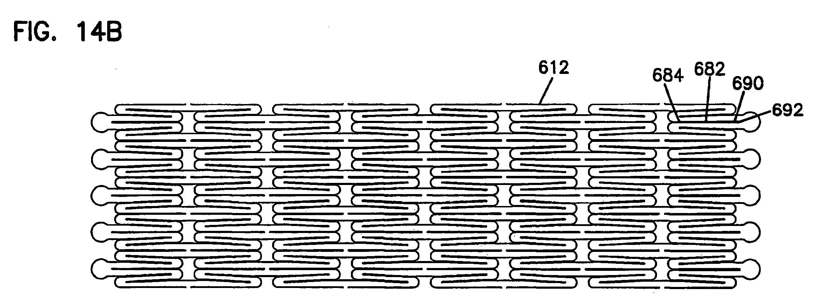

Figs. 14A and 14B illustrate a stent 612 including female interlock structures 684 in the form of longitudinal slots between or within struts. The female interlock structures 684 preferably include distally facing interlock surfaces 692 (e.g., defined by the proximal ends of the slots) located within 5 mm of a proximal end 612a of the stent 612 and within 1, 2, 3, 4 or 5 millimeters of a cell defining region of the stent. The female interlock structures 684 are sized to receive male interlock structures 682 in the form of linear posts. Preferably, the posts are connected to the outer surface of the inner tube 14 (e.g., integrally or otherwise). The posts define proximally facing interlock surfaces 690 (e.g., at the proximal ends of the posts). When the female and male interlock structures 684 and 682 are coupled as shown in Fig. 14B, the surfaces 690 and 692 engage each other to prevent distal movement of the stent 612 relative to the posts.

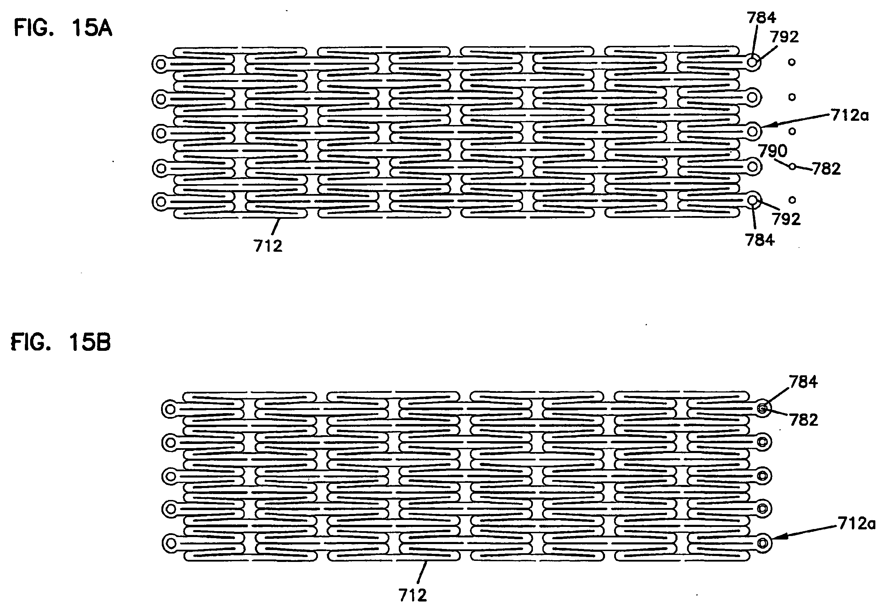

Figs. 15A and 15B illustrate a stent 712 including female interlock structures 784 in the form of circular openings defined through enlarged strut ends of the stent 712. The female interlock structures 784 preferably include distally facing interlock surfaces 792 located within 5 mm of a proximal end 712a of the stent 712 and within 1, 2, 3, 4 or 5 millimeters of a cell defining region of the stent. The female interlock structures 784 are sized to receive male interlock structures 782 in the form of cylindrical posts or pins. Preferably, the posts are connected to the outer surface of the inner tube 14 (e.g., integrally or otherwise). The posts define proximally facing interlock surfaces 790. When the female and male interlock structures 784 and 782 are coupled as shown in Fig. 15B, the surfaces 790 and 792 engage each other to prevent distal movement of the stent 712 relative to the posts.

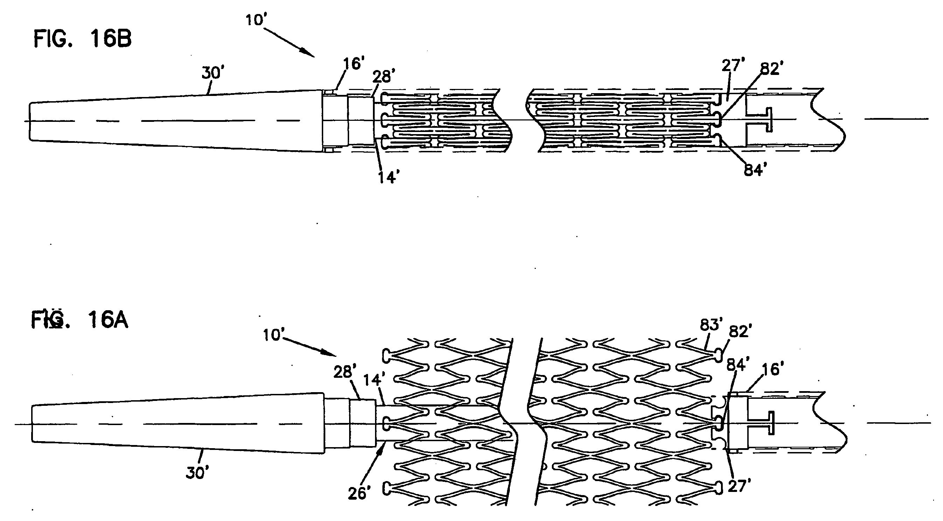

Figs. 16A and 16B show a stent delivery system 10' that is another embodiment of the present invention. The delivery system 10' includes an inner member 14' and an outer sheath 16'. The inner member 14' includes a flexible distal tip 30' and a stent mounting location 26'. Proximal and distal markers 27' and 28' are located on opposite sides of the mounting location 26'. The proximal marker 27' includes interlock structures in the form of receivers 84' or receptacles. The receivers 84' are adapted to receive and interlock with interlock structures in the form of enlargements 82' provided at the proximal end of self expanding stent 12'. The enlargements 82' are preferably within 1, 2, 3,4 or 5 millimeters of cell defining structures 83' of the stent 12'.

While the various embodiments of the present invention have related to stents and stent delivery systems, it will be appreciated that the various aspects of the present invention are also applicable to systems for delivering other types of self-expandable implants. By way of non- limiting example, other types of self-expanding implants include anastomosis devices, blood filters, grafts, vena cava filters, percutaneous valves, or other devices. Also, while it is preferred for the interlocks of the present invention to be within 5 millimeters of an end of their corresponding implant to enhance deployment control, larger spacings could be used for certain applications. Similarly, while it is preferred for the interlocks to be within 5,4,3,2 or 1 millimeters of cell defining regions of the stents, other spacings could be used in certain alternative embodiments.

It has been shown how the objects of the invention have been attained in a preferred manner.

It will be apparent to one skilled in the art that modifications may be made to the invention as described in the foregoing without departing from the scope of the claims set out below.

## Claims

1. A stent delivery system (10) comprising:
a catheter including an inner tubular member (14) having a stent attachment location (26), said inner tubular member having a proximal end (14a) and a distal end (14b);
a stent (12) mounted on the inner tubular member at the stent attachment location (26), the stent being expandable from an undeployed compressed orientation to a deployed expanded orientation, the stent including proximal and distal ends (12a, 12b), the stent also including one or more male interlock structures (82), the one or more male interlock structures being positioned at the proximal end (12a) and/or the distal end of the stent;
an outer tubular member (16) mounted on the inner tubular member, the outer tubular member being positionable in a transport position in which it covers the stent being mounted at the stent attachment location, the outer tubular member (16) also being positionable in a deploy position in which the stent is exposed;
the inner tubular member including one or more female interlock structures (84) that receive the one or more male interlock structures when the stent is in the compressed orientation to constrain axial movement of the stent relative to the inner tubular member and wherein the said one or more female interlock structures have a complementary mating geometry with respect to the male interlock structures, **characterised in that** said one or more female interlock structures (84) comprise a radiopaque material (27).

2. A stent delivery system according to claim 1, wherein the one or more male interlock structures (82) comprise rounded enlargements (88).

3. A stent delivery system according to any of claims 1-2, wherein said outer tubular member (16) has a distal end (16b) and a proximal end (16a);
wherein said proximal end (12a) of said stent (12) is releasably secured to said elongated body and is restricted from radial expansion until said distal end (16b) of said outer tubular member has been moved proximal of said stent attachment location (26) as said elongated body and said outer member move toward said deploy position.

4. A stent delivery system according to any of claims 1-3,
wherein said proximal end of said stent is releasably secured to said elongated body and is restricted from radial expansion until said distal end (16b) of said outer tubular member (16) has been moved to expose said proximal end of said stent attachment location.

5. A stent delivery system according to any of claims 1-4, wherein said one or more female interlock structures are formed in a collar (27) positioned at said stent attachment location (26).

6. A stent delivery system according to any of claims 3-4, wherein said distal end (16b) of said outer tubular member (16) has a radiopaque marking (16b').

7. A stent delivery system according to any of claims 1-6, wherein said elongated body is hollow to track over a guide wire.

8. The stent delivery system of any of claims 1-7, wherein at least a portion of the one or more male interlock structures (382) are positioned within 5 millimeters of the proximal end (312a) of the stent (312).

9. The stent delivery system of any of claims 1-8, wherein the one or more male interlock structures include a circumferential projection (88) positioned at the first end (12a) of the stent (12).

10. The stent delivery system of any of claims 1-9, wherein the one or more male interlock structures are uniformly spaced about a circumference of the stent.

11. The stent delivery system of any of claims 1-10, wherein the stent comprises an inner diameter (D1) and an outer diameter (D2), and the one or more male interlock structures (82) are located in a region defined between the inner diameter and the outer diameter.

## Patentansprüche

1. Eine Stentbeförderungseinrichtung (10), welche umfasst:
einen Katheter, der ein inneres röhrenförmiges Element (14) mit einer Stentbefestigungsstelle (26) umfasst, wobei das innere röhrenförmige Element ein proximales Ende (14a) und ein distales Ende (14b) aufweist;
einen Stent (12), der auf dem inneren röhrenförmigen Element an der Stentbefestigungsstelle (26) befestigt ist, wobei der Stent von einer komprimierten Nicht-Einsatzorientierung in eine ausgedehnte Einsatzorientierung ausgedehnt werden kann, wobei der Stent proximale und distale Enden (12a, 12b) umfasst, wobei der Stent ebenfalls eine oder mehr männliche Verriegelungsstrukturen (82) umfasst, wobei die eine oder mehr männliche Verriegelungsstrukturen am proximalen Ende (12a) und/oder dem distalen Ende des Stents positioniert sind;
ein äußeres röhrenförmiges Element (16), das auf dem inneren röhrenförmigen Element befestigt ist, wobei das äußere röhrenförmige Element in einer Transportposition positionierbar ist, in der es den Stent abdeckt, der an der Stentbefestigungsstelle befestigt ist, wobei das äußere röhrenförmige Element (16) ebenfalls in einer Einsatzposition positioniert werden kann, in der der Stent freiliegt; wobei
das innere röhrenförmige Element eine oder mehr weibliche Verriegelungsstrukturen (84) umfasst, die die eine oder mehr männlichen Verriegelungsstrukturen aufnehmen, wenn der Stent sich in der komprimierten Orientierung befindet, um die axiale Bewegung des Stents relativ zu dem länglichen Körper zu beschränken und wobei die eine oder mehr weibliche Verriegelungsstrukturen bezüglich der männlichen Verriegelungsstrukturen eine komplementäre Anschlussgeometrie haben, **dadurch gekennzeichnet, dass** die eine oder mehr weibliche Verriegelungsstrukturen (84) ein röntgenstrahlenundurchlässiges Material (27) umfasst.

2. Eine Stentbeförderungseinrichtung nach Anspruch 1, wobei die eine oder mehr männlichen Verriegelungsstrukturen (82) abgerundete Vergrößerungen (88) aufweisen.

3. Eine Stentbeförderungseinrichtung nach einem der Ansprüche 1-2, wobei das äußere röhrenförmige Element (16) ein distales Ende (16b) und ein proximales Ende (16a) aufweist;
wobei das proximale Ende (12a) des Stents (12) lösbar an dem länglichen Körper angebracht ist und an der radialen Ausdehnung gehindert wird, bis das distale Ende (16b) des äußeren röhrenförmigen Elementes proximal zur Stentbefestigungsstelle (26) bewegt wurde, während der längliche Körper und das äußere Element sich zur Einsatzposition bewegen.

4. Eine Stentbeförderungseinrichtung nach einem der Ansprüche 1-3, wobei das proximale Ende des Stents lösbar an dem länglichen Körper befestigt ist und an einer radialen Ausdehnung gehindert wird, bis das distale Ende (16b) des äußeren röhrenförmigen Elements (16) bewegt wurde, um das proximale Ende der Stentbefestigungsstelle freizulegen.

5. Eine Stentbeförderungseinrichtung nach einem der Ansprüche 1-4, wobei die eine oder mehr weibliche Verriegelungsstrukturen in einem Kragen (27) ausgebildet sind, der an der Stentbefestigungsstelle (26) positioniert ist.

6. Eine Stentbeförderungseinrichtung nach einem der Ansprüche 3-4, wobei das distale Ende (16b) des äußeren röhrenförmigen Elements (16) eine strahlenundurchlässige Markierung (16b') aufweist.

7. Eine Stentbeförderungseinrichtung nach einem der Ansprüche 1-6, wobei der längliche Körper hohl ist, um über einen Führungsdraht zu laufen.

8. Eine Stentbeförderungseinrichtung nach einem der Ansprüche 1-7, wobei zumindest ein Abschnitt der einen oder mehr männlichen Verriegelungsstrukturen (382) innerhalb von 5 Millimetern des proximalen Endes (312a) des Stents (312) positioniert werden.

9. Eine Stentbeförderungseinrichtung nach einem der Ansprüche 1-8, wobei die eine oder mehr männlichen Verriegelungsstrukturen einen Umfangsvorsprung (88) aufweisen, der am ersten Ende (12a) des Stents (12) positioniert ist.

10. Eine Stentbeförderungseinrichtung nach einem der Ansprüche 1-9, wobei die eine oder mehr männlichen Verriegelungsstrukturen gleichmäßig um einen Umfang des Stents beabstandet sind.

11. Eine Stentbeförderungseinrichtung nach einem der Ansprüche 1-10, wobei der Stent einen Innendurchmesser (D1) und einen Außendurchmesser (D2) umfasst, und die eine oder mehr männlichen Verriegelungsstrukturen (82) sich in einem zwischen dem Innendurchmesser und den Außendurchmesser definierten Bereich befinden.

## Revendications

1. Système de mise en place d'endoprothèse (10) comprenant :
un cathéter comprenant un élément tubulaire intérieur (14) comportant un emplacement de fixation d'endoprothèse (26), ledit élément tubulaire intérieur ayant une extrémité proximale (14a) et une extrémité distale (14b) ;
une endoprothèse (12) montée sur l'élément tubulaire intérieur à l'emplacement de fixation d'endoprothèse (26), l'endoprothèse pouvant être développée d'une orientation compressée non déployée à une orientation développée déployée, l'endoprothèse comprenant des extrémités proximale et distale (12a, 12b), l'endoprothèse comprenant en outre une ou plusieurs structures d'enclenchement mâles (82), lesdites structures d'enclenchement mâles étant positionnées à l'extrémité proximale (12a) et/ou à l'extrémité distale de l' endoprothèse ;
un élément tubulaire extérieur (16) monté sur l'élément tubulaire intérieur, l'élément tubulaire extérieur pouvant être positionné dans une position de transport dans laquelle il couvre l'endoprothèse montée sur l'emplacement de fixation d'endoprothèse, l'élément tubulaire extérieur (16) pouvant aussi être positionné dans une position de déploiement dans laquelle l'endoprothèse est exposée ;
l'élément tubulaire intérieur comprenant une ou plusieurs structures d'enclenchement femelles (84) qui reçoivent lesdites structures d'enclenchement mâles quand l'endoprothèse est dans l'orientation compressée pour empêcher le mouvement axial de l'endoprothèse par rapport à l'élément tubulaire intérieur et dans lequel lesdites structures d'enclenchement femelles ont une géométrie conjuguée complémentaire par rapport aux structures d'enclenchement mâles, **caractérisé en ce que** lesdites structures d'enclenchement femelles (84) comprennent un matériau radio-opaque (27).

2. Système de mise en place d'endoprothèse selon la revendication 1, dans lequel lesdites structures d'enclenchement mâles (82) comprennent des protubérances arrondies (88).

3. Système de mise en place d'endoprothèse selon l'une quelconque des revendications 1 à 2, dans lequel ledit élément tubulaire extérieur (16) a une extrémité distale (16b) et une extrémité proximale (16a) ;
dans lequel ladite extrémité proximale (12a) de ladite endoprothèse (12) est fixée de manière amovible audit corps allongé et est empêchée de se développer radialement jusqu'à ce que ladite extrémité distale (16b) dudit élément tubulaire extérieur ait été déplacée à proximité dudit emplacement de fixation d'endoprothèse (26) lorsque ledit corps allongé et ledit élément extérieur vont vers ladite position déployée.

4. Système de mise en place d'endoprothèse selon l'une quelconque des revendications 1 à 3, dans lequel ladite extrémité proximale de ladite endoprothèse est fixée de manière amovible audit corps allongé et est empêchée de se développer radialement jusqu'à ce que ladite extrémité distale (16b) dudit élément tubulaire extérieur (16) ait été déplacée pour exposer ladite extrémité proximale dudit emplacement de fixation d'endoprothèse.

5. Système de mise en place d'endoprothèse selon l'une quelconque des revendications 1 à 4, dans lequel lesdites structures d'enclenchement femelles sont formées dans un collier (27) positionné audit emplacement de fixation d'endoprothèse (26).

6. Système de mise en place d'endoprothèse selon l'une quelconque des revendications 3 à 4, dans lequel ladite extrémité distale (16b) dudit élément tubulaire extérieur (16) comporte un repère radio-opaque (16b').

7. Système de mise en place d'endoprothèse selon l'une quelconque des revendications 1 à 6, dans lequel ledit corps allongé est creux pour suivre un fil de guidage.

8. Système de mise en place d'endoprothèse selon l'une quelconque des revendications 1 à 7, dans lequel au moins une partie des structures d'enclenchement mâles (382) sont positionnées à une distance maximale de 5 millimètres de l'extrémité proximale (312a) de l'endoprothèse (312).

9. Système de mise en place d'endoprothèse selon l'une quelconque des revendications 1 à 8, dans lequel les structures d'enclenchement mâles comprennent une saillie périphérique (88) positionnée à la première extrémité (12a) de l'endoprothèse (12).

10. Système de mise en place d'endoprothèse selon l'une quelconque des revendications 1 à 9, dans lequel les structures d'enclenchement mâles sont régulièrement espacées autour d'une circonférence de l'endoprothèse.

11. Système de mise en place d'endoprothèse selon l'une quelconque des revendications 1 à 10, dans lequel l'endoprothèse comprend un diamètre intérieur (D1) et un diamètre extérieur (D2), et les structures d'enclenchement mâles (82) sont situées dans une région définie entre le diamètre intérieur et le diamètre extérieur.
